# EUROPÄISCHE PATENTANMELDUNG

(11) **EP 1 391 175 A2**
(43) Veröffentlichungstag der Anmeldung: **25.02.2004**
(21) Anmeldenummer: 03018242.2
(22) Anmeldetag: 11.08.2003
(51) Int. Cl.: A61B 1/24, A61C 1/08

(54) **Arztliches oder zahnärztliches stabförmiges Handstück mit einem Display**

(30) Priorität: 22.08.2002 DE 10238556
(71) Anmelder: Kaltenbach & Voigt GmbH & Co. KG, 88400 Biberach (DE)
(72) Erfinder: Schmid, Gerhard, 88441 Mittelbiberach (DE); Löhn, Gerd, 88400 Biberach-Rissegg (DE); Liebhardt, Franz, 88416 Ochsenhausen (DE); Mösslang, Hubert, 89610 Oberdischingen (DE)
(74) Vertreter: Schmidt-Evers, Jürgen, Dipl.-Ing.

(57) **Zusammenfassung**

Die Erfindung betrifft ein medizinisches oder zahnmedizinisches Handstück (1). Um die Bedienbarkeit des Handstücks und/oder die Durchführbarkeit der Behandlung zu verbessern und insbesondere zu vereinfachen, weist das Handstück (1) ein Display (21) zur Anzeige von arbeitsrelevanten Daten oder Bildern auf.

## Beschreibung

Die Erfindung betrifft ein ärztliches oder zahnärztliches stabförmiges Handstück nach dem Oberbegriff des Anspruches 1.

Es ist der Zweck eines solchen Handstücks, mit einem im vorderen Endbereich des stabförmigen Handstücks angeordneten Funktionselement mittelbar oder unmittelbar auf eine Behandlungsstelle am menschlichen oder tierischen Körper einzuwirken. Um eine visuelle Beobachtung der Behandlungsstelle und/oder des Funktionselementes durch den Behandler möglichst wenig zu beeinträchtigen, ist das Funktionselement im vorderen Endbereich eines länglichen Halsabschnitts angeordnet, von dem sich ein Griffabschnitt nach hinten erstreckt, der dem manuellen Ergreifen des Handstücks dient. Diese Ausgestaltung ermöglicht es außerdem, eine Behandlungsstelle in einer Körperhöhle zu erreichen, wie es bei einem zahnärztlichen Handstück im Mundraum des Patienten der Fall ist, wobei die Länge des Halsabschnitts an die Tiefe oder gewünschte Tiefe der Körperhöhle angepasst ist oder länger sein kann.

Bei der vorbeschriebenen Bauweise des Handstücks handelt es sich um eine übliche Bauweise, die z.B. im DE 297 20 616 U1 und in der DE 100 43 749 A1 beschrieben ist.

Bei einem Handstück der vorliegenden Art handelt es sich im weiteren um einen Gegenstand, mit dem mit unterschiedlichen Funktionsmerkmalen auf den Körper eingewirkt werden kann, die von der Bedienungsperson vorbestimmbar oder auch während der Behandlung veränderlich sind, z.B. die Drehzahl eines Drehwerkzeugs. Der Behandler hat deshalb seine Aufmerksamkeit nicht nur auf die Behandlung zu richten, sondern auch auf die Funktionseinstellung des Handstücks, die einen wesentlichen Einfluss auf die Behandlung nehmen kann.

Im vorliegenden Fachbereich sind für sich baulich und/oder funktionell voneinander unterscheidende Handstückarten besondere Fachbezeichnungen eingeführt worden. Man unterscheidet zwischen Bearbeitungshandstücken, die dazu dienen, mittels eines insbesondere bewegbaren Werkzeugs, z.B. spanabhebend, auf den Körper einzuwirken, und sogenannten Funktionshandstücken, die dazu dienen, Hilfsmittel zur Behandlungsstelle zuzuführen oder abzuführen, z.B. Wasser, Luft bzw. ein Wasser-Luft-Gemisch oder Licht, und Sondenhandstücken, die dazu dienen, eine Behandlungsstelle z.B. hinsichtlich ihrer Beschaffenheit zu sondieren, wobei es sich um ein Handstück mit einer Bildaufnahmevorrichtung handeln kann, mit der Bilder von der Behandlungsstelle aufgenommen und wiedergegeben werden können, z.B. auf einem dem Behandlungsstuhl bzw. dem Behandlungsplatz zugeordneten Bildschirm.

Der Erfindung liegt deshalb die Aufgabe zugrunde, bei einem Handstück der eingangs angegebenen Art die Bedienbarkeit des Handstücks und/oder die Durchführbarkeit der Behandlung zu verbessern und insbesondere zu vereinfachen.

Das Handstück soll so ausgebildet sein, dass die vom Behandler aufzubringende Aufmerksamkeit zur Überwachung der Funktion des Handstücks verringerbar ist und somit der Behandler seine Aufmerksamkeit vermehrt auf die Behandlung des Körpers richten kann.

Diese Aufgabe wird durch die Merkmale des Anspruches 1 gelöst. Vorteilhafte Weiterbildungen der Erfindung sind in den Unteransprüchen beschrieben.

Bei der erfindungsgemäßen Ausgestaltung ist am Handstück ein Display angeordnet, auf dem Funktionsmerkmale bzw. arbeitsrelevante Daten des Handstücks und/oder Bilder der Behandlungstabelle anzeigbar sind. Hierbei kann es sich um ein oder mehrere jeweils in Funktion befindliche Daten und/oder einstellbare Daten handeln.

Hierdurch wird die vom Behandler auf die Funktion des Handstücks zu richtende Aufmerksamkeit wesentlich verringert, weil der Behandler die in Funktion befindlichen oder einstellbaren Funktionsmerkmale am Display erkennen und kontrollieren kann und deshalb seine Aufmerksamkeit vermehrt auf die Behandlung am Körper richten kann.

Bei einem Arbeitshandstück kann es sich bei dem wenigstens einen auf dem Display anzeigbaren Funktionsmerkmal z.B. um die Arbeitsgeschwindigkeit, insbesondere Drehzahl, eines Bearbeitungswerkzeugs handeln. Beim Funktionshandstück kann es sich bei dem auf dem Display anzeigbaren wenigstens einen Funktionsmerkmal um die einstellbare oder eingestellte Art eines Behandlungsmediums, z.B. Luft, Wasser oder Spray, handeln. Der Behandler braucht sich diese Daten nicht zu merken. Er kann deshalb seine Aufmerksamkeit vermehrt auf die Behandlung des Körpers richten. Um ein Funktionsmerkmal einzustellen oder zu kontrollieren, welches Funktionsmerkmal in Funktion ist, braucht der Behandler lediglich einen Blick auf das Display zu werfen, wodurch er ergonomisch günstig die von ihm gewünschte Information erhält.

Die erfindungsgemäße Ausgestaltung eignet sich auch für ein Sondenhandstück oder ein kombiniertes Arbeits/Sondenhandstück oder ein kombiniertes Funktions/Sondenhandstück mit einer im vorderen Bereich des Halsabschnitts angeordneten Bildaufnahmevorrichtung, deren Bilder auf dem Display anzeigbar sein. Hierdurch erhält der Behandler eine direkte Sicht auf die auf dem Display abgebildete Behandlungsstelle bzw. die aufgenommenen Bilder. Dies gilt auch für solche Behandlungsstellen, die bisher nur mittels Spiegel eingesehen werden konnten, was für den Behandler ergonomisch aufwendig und anstrengend ist, wobei auch mit beträchtlichen Einbußen der Sicht auf die Behandlungsstelle zu rechnen ist.

Es ist zwar aus der DE 40 09 438 A1 bereits bekannt, die von einer Bildaufnahmevorrichtung an einem Handstück aufgenommenen Bilder auf einem Display anzuzeigen, jedoch ist bei diesem Stand der Technik das Display einem Behandlungsstuhl im Bereich eines Steuerpultes zugeordnet. Bei dieser vorbekannten Ausgestaltung muss der Behandler verhältnismäßig große Bewegungen mit dem Kopf oder seinem Körper ausführen, um seinen Blick auf das Display zu richten. Hierbei besteht die Gefahr, dass dann, wenn das Handinstrument an der Behandlungsstelle verbleibt, der Behandler das Handinstrument nicht mehr kontrollieren und/oder positionieren kann und das Handstück unbeabsichtigt bewegte wodurch falsche Handstückbewegungen ausgeführt werden können und ggf. sogar Verletzungen des Körpers im Bereich der Behandlungsstelle zugefügt werden können. Dagegen ermöglicht es die erfindungsgemäße Ausgestaltung, das Handstück beim Blick auf das Display manuell zu kontrollieren, wodurch nicht nur die vorbeschriebenen Schwierigkeiten vermieden werden, sondern auch eine einfachere und präzisere Handstückführung möglich ist.

Eine vorteilhafte Anordnungsstelle für das Display befindet sich im Bereich zwischen dem Griffabschnitt und dem Halsabschnitt. Hierbei kann das Handstück sowohl gerade als auch bogenförmig gekrümmt oder winkelförmig ausgebildet sein, wobei der Halsabschnitt mit einem spitzen Winkel seitlich abgebogen oder abgewinkelt sein kann.

Ein Funktionshandstück wird vom Behandler zur Ausübung einer Funktion an der Behandlungsstelle manuell so ergriffen, dass der zwischen dem z.B. seitlich ausgebogenen oder abgewinkelten Halsabschnitt und der Mittelachse des Griffabschnitts eingeschlossene Winkel zur dem Behandler abgewandten Seite hin offen ist, d.h. der z.B. zu seinem freien Ende hin konvergent ausgebildete Halsabschnitt ist dabei in die dem Behandler abgewandte Richtung gerichtet. Es ist deshalb vorteilhaft, das Display auf der Seite des Handstücks anzuordnen, die der Ausbiegung bzw. Abwinklung entgegengesetzt ist. In diesem Bereich hat der Behandler einen direkten oder günstigen Blick auf das Display, so dass er die abgebildeten arbeitsrelevanten Daten deutlich ablesen kann.

Dagegen ist bei einem seitlich ausgebogenen oder abgewinkelten Bearbeitungshandstück der mit dem Halsabschnitt vergleichbare vordere seitlich ausgebogene oder abgewinkelte Schenkel zu der eine Einstecköffnung für ein Werkzeug am Kopf des Handstücks abgewandten Seite hin abgewinkelt oder abgebogen. Bei einem solchen Handstück es deshalb vorteilhaft, das Display im Bereich des Freiraumes des Handstücks anzuordnen, der durch die einen stumpfen Winkel zwischen sich einschließenden Handstücksabschnitten (Griffabschnitt, Schenkel- oder Halsabschnitt) begrenzt ist, insbesondere im Bereich des Scheitels der Abwinklung bzw. im Übergangsbereich zwischen dem geraden und dem gekrümmten Handstückabschnitt. Hierbei kann das Display bezüglich der Abwinklungsebene zu einer Umfangsrichtung hin versetzt angeordnet sein, weil in dieser Position der Behandler eine noch bessere direkte Sicht auf das Display hat. Allerdings ergeben sich bei einer solchen Ausgestaltung unterschiedliche Handstücke für Rechts- und Linkshänder. Es ist deshalb diesbezüglich vorteilhaft, das Display in der Abwinklungs- bzw. Krümmungsebene anzuordnen. Weitere Ausgestaltungsmerkmale der Erfindung tragen dazu bei, die direkte Sicht auf das Display zu verbessern und eine einfache und kostengünstige Herstellung zu erreichen.

Die erfindungsgemäße Ausgestaltung mit dem Display ist insbesondere in Kombination mit einer Bildaufnahmevorrichtung vorteilhaft, die im vorderen Endbereich des Handstücks angeordnet ist, und deren Bilder auf dem Display anzeigbar sind. Bei dieser Ausgestaltung können die von der Behandlungsstelle aufgenommenen Bilder bei Gewährleistung der vorbeschriebenen Vorteile handhabungsfreundlich und ergonomisch günstig abgelesen werden.

Die erfindungsgemäße Bildaufnahmevorrichtung nach Anspruch 11 und folgende eignet sich jedoch nicht nur in Kombination mit einem am Handstück angeordneten Display nach einem der Ansprüche 1 bis 10, sondern die Bildaufnahmevorrichtung eignet sich auch in Kombination mit einem Display, das im Bereich einer Versorgungseinrichtung für das Handstück angeordnet ist, mit dem das Handstück durch eine flexible Versorgungsleitung verbunden ist, oder die Bildaufnahmevorrichtung eignet sich auch in Kombination mit einem Display, das im Bereich des Behandlungsplatzes vom Behandler sichtbar angeordnet ist.

Da ein vorliegendes Handstück zum einen von kleiner Bauweise ist, und zum anderen bewegbar angeordnet ist, und deshalb Raumknappheit vorliegt, eignet sich eine drahtlose Übertragung der auf dem Display des Handstücks anzuzeigenden Daten sehr vorteilhaft. Im Falle von auf dem Display anzuzeigenden arbeitsrelevanten Bildern sind einer Bildaufnahmevorrichtung im vorderen Endbereich des Handstücks eine Umwandlungsvorrichtung zum Umwandeln der Bilder in übertragbare Daten und ein Datensender zugeordnet, der es ermöglicht, die Bilddaten drahtlos einem Datenempfänger des Handstücks zu übertragen. Die übertragenen Daten werden durch eine zweite Datenumwandlungsvorrichtung so umgewandelt, dass sie auf dem Display als Bilder dargestellt werden können.

Im Falle der Anzeige von arbeitsrelevanten Daten auf dem Display des Handstücks werden dem Datenempfänger arbeitsrelevante Daten drahtlos, z. B. von einem Datensender der Versorgungseinrichtung des zugehörigen Behandlungsstuhls, übertragen, die von der Datenumwandlungsvorrichtung zu auf dem Display anzeigbaren arbeitsrelevanten Daten umgewandelt werden.

Nachfolgend werden vorteilhafte Ausgestaltungen der Erfindung anhand mehrerer Ausführungsbeispiele und Zeichnungen näher erläutert. Es zeigen:
Fig. 1 ein erfindungsgemäßes Handstück in perspektivischer Darstellung;
Fig. 2 das Handstück in der Seitenansicht;
Fig. 3 ein Handstück in abgewandelter Ausgestaltung in der Seitenansicht;
Fig. 4 ein Handstück in weiter abgewandelter Ausgestaltung in der Seitenansicht;
Fig. 5 bis 7 Draufsichten auf ein Handstück-Display bei Abbildung unterschiedlicher Daten oder Bilder.

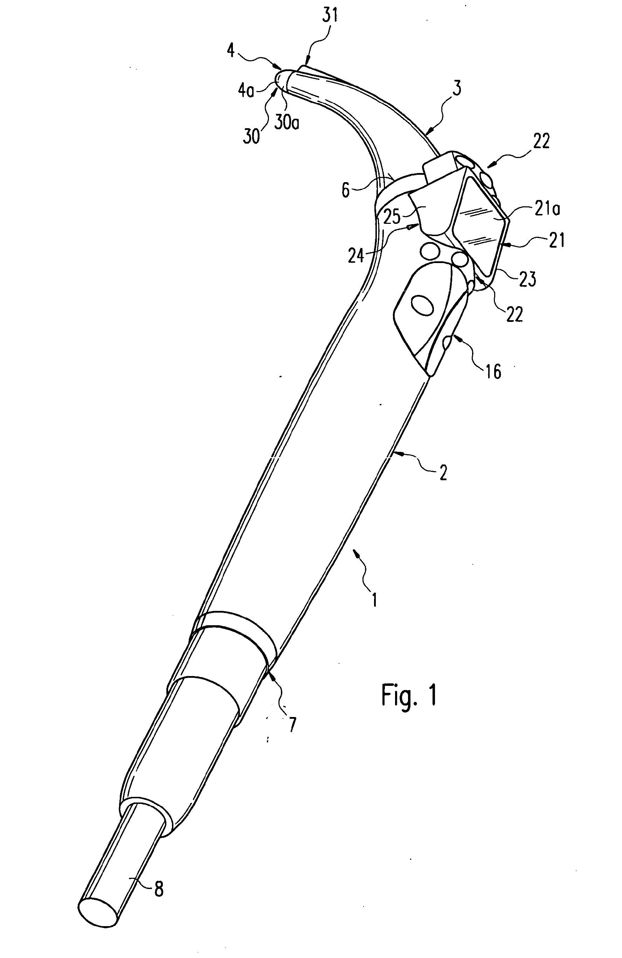

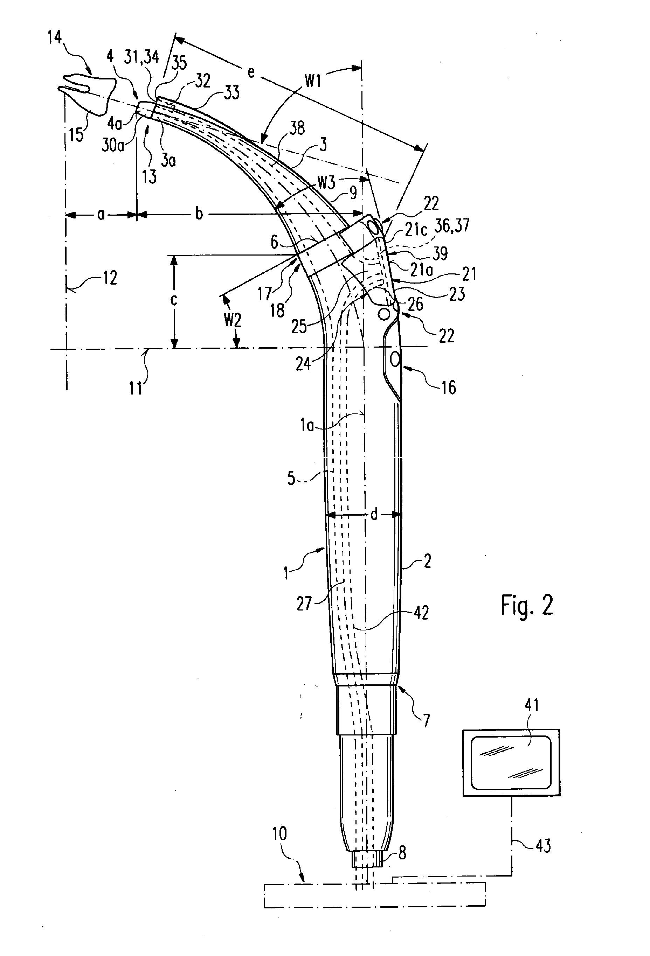

Das in seiner Gesamtheit mit 1 bezeichnete Handstück ist gemäß Fig. 1 und 2 ein sog. Funktionshandstück zur Zuführung von einem oder mehreren Behandlungsmedien, z.B. Wasser, Luft bzw. Spray und/oder Licht. Das Handstück 1 ist von länglicher stabförmiger Bauform und es weist einen stabförmigen Griffabschnitt 2 auf, von dem sich ein Halsabschnitt 3 nach vorne erstreckt, der in seinem vorderen Endbereich ein Funktionselement 4 zum direkten oder indirekten Behandeln des menschlichen oder tierischen Körpers aufweist, z.B. im vorliegenden Fall eine Auslassdüse 4a für ein oder mehrere Behandlungsmedien, die der Auslassdüse 4a durch eine oder mehrere Zuführungsleitungen 5 zugeführt werden, die sich längs durch das Handstück 1 erstrecken und von denen beispielsweise eine Zuführungsleitung 5 dargestellt ist. Hierzu sind der Griffabschnitt 2 und der Halsabschnitt 3 hülsenförmig ausgebildet, so dass sich darin Freiräume für die Durchführung befinden.

Der Griffabschnitt 2 und der Halsabschnitt 3 können einteilig oder mit einer Fuge 6 zweiteilig ausgebildet und miteinander verbunden sein, wobei sie lösbar oder unlösbar miteinander verbunden sein können. Der Halsabschnitt 3 bildet vorzugsweise eine Kanüle.

Am hinteren Ende des Griffabschnitts 2 ist ein Verbindungsteil 7 z.B. einteilig angeformt, das dazu dient, das Handstück 1 mit einem flexiblen Versorgungsschlauch 8 zu verbinden, der sich von einer angedeuteten Versorgungseinrichtung 10 erstreckt und der Funktionssteuerung des Handstücks 1 dient. Das Verbindungsteil 7 kann z. B. ein Anschlussrohrstutzen sein, mit dem der Versorgungsschlauch 8 verbunden ist, z.B. durch einen Schraubanschluss.

Das Handstück 1 ist in seinem vorderen Bereich seitlich gekrümmt oder abgewinkelt. Der Beginn der vorzugsweise kreisbogenförmigen Krümmung 9 weist vorzugsweise die Form etwa eines Viertelkreises auf. Der Beginn der Krümmung ist durch eine Radiallinie 11 gekennzeichnet, die zugleich den Beginn eines Quadranten darstellt, in dem die Krümmung 9 verläuft. Die andere den Quadranten begrenzende Radiallinie läuft etwa parallel zur Mittelachse 1a des Handstücks 1 im Bereich des Griffabschnitts 2. Wie insbesondere aus Fig. 2 deutlich zu entnehmen ist, endet der Kopf 13 des Handstücks 1 vor der zweiten Radiallinie 12, wobei dieser Abstand a vorzugsweise etwa 5 mm bis 30 mm, insbesondere etwa 15 mm, beträgt. Hierbei kann die Längsmittelachse 1a im Bereich des Kopfes 13 mit der gedachten, sich gerade erstreckenden Mittelachse 1a im Bereich des Griffabschnitts 2 einen nach vorne offenen spitzen Winkel W1 von etwa 70° bis 90°, insbesondere etwa 80° bis 85°, betragen.

Der seitliche Abstand b, den der Kopf 13 von der gedachten Verlängerung der geraden Längsmittelachse 1a des Griffabschnitts 2 aufweist, kann etwa 30 bis 60 mm, insbesondere etwa 45 mm, betragen.

Um im Betrieb des Handstücks 1 den Blick des Behandlers auf die Behandlungsstelle 14, die z.B. durch einen Zahn 15 verdeutlicht ist, möglichst wenig zu beeinträchtigen, ist angestrebt, die Querschnittsgröße des Kopfes 13 möglichst klein auszubilden. Beim Ausführungsbeispiel verjüngt sich deshalb der Halsabschnitt 3 nach vorne, vorzugsweise kontinuierlich, wobei die Querschnittsgröße der - z.B. durch Ein- oder Aufschrauben befestigten Auslassdüse 4a - im wesentlichen der Querschnittsgröße des Halsabschnittsendes 3a entsprechen kann. Beim Ausführungsbeispiel ist das Handstück 1 im Bereich des Griffabschnitts 2 und im Bereich des Halsabschnitts 3 rund, insbesondere kreisrund ausgebildet. Das Material des Griffabschnitts 2 und des Halsabschnitts 3, die im wesentlichen hülsenartig ausgebildet sind, kann aus korrosionsfestem Metall, z.B. legiertem Stahl oder Kunststoff, bestehen.

Das Handstück 1 weist an einer handhabungsfreundlich erreichbaren Betätigungsstelle ein oder mehrere Betätigungselemente 16 auf, durch deren Betätigung es möglich ist, Funktionsmerkmale des Handstücks 1 zu verändern, z.B. Ein- und Auszuschalten, hier z.B. die Ein- und Ausschaltung von wenigstens einem Behandlungsmedium.

Das oder die Betätigungselemente 16 sind auf der dem seitlichen Versatz des Kopfes 13 abgewandten Seite des Handstücks 1 im wesentlichen an der Mantelfläche des Handstücks 1 angeordnet und z.B. durch wenigstens eine Drucktaste gebildet. Das oder die Betätigungselemente 16 durchsetzen den Mantel des Handstücks 1 bzw. Griffabschnitts 2 und können z.B. mit wenigstens einem Ventil in der oder den Zuführungsleitungen 5 in Antriebsverbindung stehen.

Beim Ausführungsbeispiel befindet sich die Teilungsfuge 6 in einem nach vorne versetzten Abstand c vom Scheitel der Abwinkelung oder vom Beginn der Krümmung 9. Der Winkel W2 zwischen der Radiallinie 11 und der sich vorzugsweise rechtwinklig zur Längsmittelachse 1a erstreckenden Teilungsfuge 6 kann bis etwa 45°, vorzugsweise bis etwa 30°, betragen. Die mechanische Verbindung 17 zwischen dem Griffabschnitt 2 und dem Halsabschnitt 3 ist vorzugsweise eine Steckverbindung, insbesondere eine mit einer Verrastungsvorrichtung 18 kombinierte Steckverbindung. Eine solche Verbindung 17 kann ohne ein relatives Drehen der Teile zueinander montiert oder gelöst werden, insbesondere nur werkstattmäßig, so dass der Behandler mit den ihm in der Praxis normalerweise zur Verfügung stehenden Mitteln nicht in der Lage ist, die Verbindung 17 zu lösen. Hierdurch sollen falsche Behandlungen des Handstücks 1 vermieden werden.

Am Handstück 1 ist ein Display 21 angeordnet, an dem arbeitsrelevante Daten oder Bilder des Handstücks oder der Behandlungsstelle 14 anzeigbar sind, und das an der Mantelfläche des Handstücks 1 oder in davon etwas abgehobener Position angeordnet ist. Die Displayfläche 21a ist vorzugsweise eben oder leicht gekrümmt, und sie stellt sich bei einem im Querschnitt etwa runden Handstück als eine Tangente oder Linie dar, die bezüglich der Mantelfläche 9 des Handstücks einen nach außen gerichteten radialen Abstand aufweisen kann. Die Breite des Displays 21 oder seiner Fläche 21a entspricht in etwa der Querschnittsgröße d des Handstücks 1 in dem Bereich, in dem das Display 21 angeordnet ist.

Eine vorteilhafte Anordnungsstelle für das Display 21 ist der vordere Bereich des Griffabschnitts 2, so dass das Display 21 einen Abstand e vom Kopf 13 aufweist, der die Zugänglichkeit zur Behandlungsstelle 14 und in einer Körperhöhle gewährleistet, z.B. im Mundraum eines Patienten, wobei der Abstand e gleich oder größer ist als die Körperhöhlentiefe, so dass in der Betriebsstellung des Handstücks 1 das Display 21 sich außerhalb der Körperhöhle befindet. Beim Ausführungsbeispiel ist der vordere Bereich des Griffabschnitts 2 im Bereich des Abstands c bereits im Sinne der Krümmung gekrümmt, wobei das Display 21 in diesem Krümmungsbereich angeordnet ist.

Um Rechts- und Linkshändern mit ein und derselben Ausgestaltung Rechnung zu tragen, ist es vorteilhaft, das Display 21 bezüglich der die Abwinklung oder Krümmung des Handstücks 1 enthaltenden Längsebene E1 symmetrisch anzuordnen, wie es das Ausführungsbeispiel zeigt.

Dem Display 21 ist wenigstens ein manuell zugängliches Betätigungselement 22 vorzugsweise am Handstück 1 zugeordnet, um z.B. das Display 21 ein- und auszuschalten und/oder eine bestimmte von mehreren vorhandenen Anzeigen auszuwählen. Eine bevorzugte Anordnungsstelle für das oder die Betätigungselemente 22 ist der Randbereich des Displays 21. Beim Ausführungsbeispiel sind ein oder mehrere Betätigungselemente 22 vorzugsweise direkt hinter und/oder vor dem Display 21 angeordnet, vorzugsweise jeweils in einer Querreihe, z.B. jeweils drei Stück. Die Form der Displayfläche 21a ist vorzugsweise viereckig, z.B. quadratisch, und ggf. mit gerundeten Ecken oder insgesamt rund. Sofern dem Display 21 weitere Betätigungselemente zugeordnet werden sollen, die hinten oder am hinteren Rand keinen Platz haben, können ein oder mehrere Betätigungselemente 22 auch am vorderen Rand des Displays 21 bzw. vor diesem, angeordnet sein, wie es das Ausführungsbeispiel ebenfalls zeigt. Auch bei dieser Ausgestaltung eignet sich ein Handstück 1 zumindest als Vorfertigungsbauteil sowohl für Rechts- als auch Linksausführung.

Das Display 21 weist einen scheibenförmigen Abdeckkörper 21c auf, der in einer rahmenförmigen Fassung 23 gehalten und von außen montierbar bzw. demontierbar ist. Die Fassung 23 ist an einem Sockel 24 ausgebildet, der drei oder vier von der Mantelfläche 9 des Handstücks 1 abstehende, einen Rahmen bildende Seitenwände 25 aufweisen kann, in deren freien Randbereichen die Fassung 23 angeordnet sein kann. Im von den Seitenwänden 25 begrenzten Hohlraum ist eine Leitungsdurchführung 26 für wenigstens eine Leitung zur Zuführung von Energie oder von Steuer- oder Funktionssignalen des Handstücks 1 angeordnet. Die wenigstens eine Leitung 27 kann sich von hinten längs durch das Handstück 1 erstrecken, z.B. vom Steuergerät 10 her durch den Versorgungsschlauch 8.

Das Display 21 kann mit der Mittelachse 1a einen spitzen Winkel W3 einschließen, der nach vorne offen ist. Wenn das Display 21 im Bereich der Krümmung 9 (Fig. 1) angeordnet ist, ist der Winkel W3 kleiner als der Winkel W1, wobei er etwa die Hälfte betragen kann.

Das Display 21 eignet sich sehr vorteilhaft in Kombination mit einer Bildaufnahmevorrichtung 31 zur Aufnahme von Bildern der Behandlungsstelle 14, wobei die Bilder auf dem Display 21 abgebildet werden können. Hierbei kann wenigstens ein funktionsrelevantes Merkmal des Handstücks, z.B. die jeweils vorhandene Drehzahl, weiterhin auf dem Display 21 angezeigt werden, z.B. in dessen Randbereich, wo es die Bilddarstellung nicht stört.

Beim Ausführungsbeispiel ist die Bildaufnahmevorrichtung 31 im vorderen Endbereich des Handstücks 1 angeordnet, wobei sie am Halsabschnitt 3 angeordnet sein kann, z.B. in einer bezüglich der Mittelachse 1a seitlich versetzten Position. Beim Ausführungsbeispiel ist die Bildaufnahmeeinrichtung 31 in der Abwinklungs- bzw. Krümmungsebene E1 angeordnet und z. B. bezüglich des Halsabschnitts 3 nach vorne versetzt angeordnet, z.B. als Bildaufnahmemodul 32 in einem Materialansatz 33, der sich z. B. wulstförmig nach hinten erstrecken kann und im Bereich des Halsabschnitts 3 größerer Dicke in den Halsabschnitt 3 auslaufen kann.

Die Bildaufnahmevorrichtung 31 weist eine Lichteinlassöffnung 34 auf, die durch eine lichtdurchlässige Verschlussscheibe 35, insbesondere aus Glas oder Kunststoff, abgedeckt ist. Hinter der Verschlussscheibe ist eine Bildumwandlungsvorrichtung 36 angeordnet, die das aufgenommene Bild in elektronische Daten umwandelt. Diese Daten werden einer Bildwiedergabevorrichtung 37 übermittelt, von der das Display 21 ein Teil ist, und die die Daten auf dem Display 21 jeweils als Bild sichtbar wiedergibt.

Es ist möglich, die Bildumwandlungsvorrichtung 36 der Bildwiedergabevorrichtung 37 zuzuordnen und die aufgenommenen Bilder durch einen sich längs durch den Halsabschnitt 3 und in den Griffabschnitt 2 sowie durch die Leitungsdurchführung 26 erstreckenden Lichtleiter 38 der Bildumwandlungsvorrichtung 36 zuzuleiten. Es ist auch möglich, die Bildumwandlungsvorrichtung 36 der Bildaufnahmevorrichtung 31 zuzuordnen und die elektronischen Daten der aufgenommenen Bilder durch eine den Lichtleiter 38 ersetzende Signal- oder Datenleitung der Bildwiedergabevorrichtung 37 zuzuführen.

Der Bildwiedergabevorrichtung 37 ist eine elektronische Steuervorrichtung 39 zugeordnet, die es ermöglicht, unterschiedliche Bildwiedergabefunktionen einzuschalten, z. B ein jeweiliges Standbild und/oder einen Bildschirm 41, der im Bereich eines zugehörigen Behandlungsstuhls angeordnet ist und durch eine Datenleitung 42, 43 durch die Versorgungseinrichtung 10 mit der Bildwiedergabevorrichtung 37 verbunden ist. Zur Einschaltung dieser Steuerungsvorgänge können ein oder mehrere der beschriebenen Betätigungselemente 22 vorgesehen sein.

Dem Handstück 1 ist vorzugsweise eine Beleuchtungsvorrichtung 30 zum Beleuchten der Behandlungsstelle 14 zugeordnet, die ein im vorderen Endbereich des Handstücks 1 oder der Kanüle angeordnetes Lichtabstrahlelement 30a aufweist; das durch den Düsenkörper 4 gebildet sein kann und aus lichtdurchlässigem Material besteht, z. B. aus Kunststoff oder Glas, und dem das Licht von hinten zugeführt wird, z.B. durch einen sich längs im Handstück 1 oder Halsabschnitt 3 erstreckenden Lichtleiter. Das Lichtabstrahlelement 30a ist vorzugsweise durch eine Steckverbindung oder Schnellverbindung am Handstück 1 oder Halsabschnitt 3 lösbar befestigt und insbesondere durch ein Ersatzstück austauschbar. Das Lichtabstrahlelement 30a bildet einen vorderen Längsabschnitt des Handstücks 1 bzw. Halsabschnitts und ist geeignet, eine Behandlungsstelle 14 räumlich zu beleuchten.

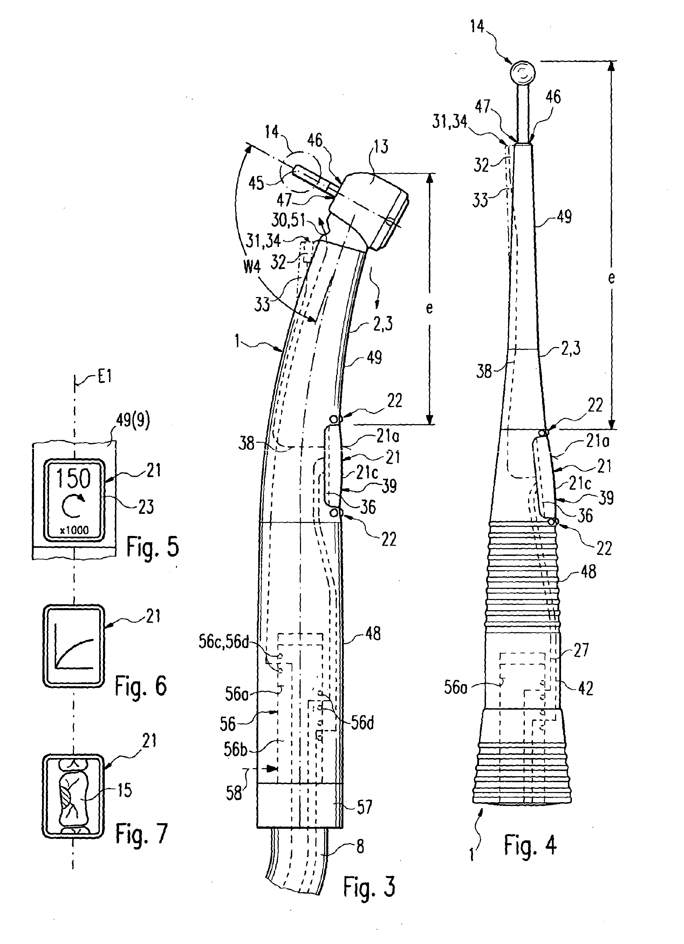

Das Ausführungsbeispiel gemäß Fig. 3, bei dem gleiche oder vergleichbare Teile mit gleichen Bezugszeichen versehen sind, zeigt ein Handstück 1 in Form eines Arbeitshandstücks mit einem auf den Körper einwirkbaren Werkzeug 45, hier ein Bohrer oder Fräser, der lösbar in einer im Kopf 13 angeordneten Haltevorrichtung 46 gehalten ist, die eine seitliche Einstecköffnung 47 für das Werkzeug 45 aufweist. Dieses Handstück ist zu der Einstecköffnung 47 abgewandten Seite gekrümmt oder abgewinkelt, z.B. in seiner vorderen Hälfte, wie es an sich bekannt ist. Bei einem solchen Handstück 1 ist das Display 21 auf der Seite des Handstücks 1 angeordnet, die der Einstecköffnung 47 abgewandt ist, wobei das Display 21 ebenfalls den Abstand e vom vorderen Ende des Handstücks 1 aufweist und vorzugsweise im mittleren Längsbereich des Handstücks 1, z.B. im Übergangsbereich zwischen einem geraden hinteren Handstückabschnitt 48 und dem vorderen Handstückabschnitt 49 angeordnet ist, der beim vorliegenden Ausführungsbeispiel gekrümmt ist oder auch im vorbeschriebenen Sinne abgewinkelt sein kann, sich in seiner Querschnittsgröße nach vorne kontinuierlich verjüngt und einen Griffabschnitt bildet.

Auch beim Ausführungsbeispiel nach Fig. 3 kann im vorderen Endbereich des vorderen Handstückabschnitts 49 eine Bildaufnahmevorrichtung 21 angeordnet sein, die hier vorzugsweise auf der der Einstecköffnung 47 zugewandten Seite des Handstückabschnitts 49 angeordnet ist und in einen Materialansatz 33 integriert, z.B. als Bildaufnahmemodul darin eingesetzt sein kann. In dieser Position ergibt sich aufgrund des dreieckigen Freiraums zwischen dem Werkzeug 45 und dem Handstück 1 eine günstige Position der Bildaufnahmevorrichtung 31 bezüglich der Behandlungsstelle 14. Die Bildaufnahmevorrichtung 31 kann vor oder vorzugsweise hinter einer angedeuteten Medienauslassöffnung 51 für Wasser, Luft oder Spray und/oder einer Lichtaustrittsöffnung der Beleuchtungseinrichtung 30 angeordnet sein.

Beim Ausführungsbeispiel ist das Display 21 im hinteren Bereich des gekrümmten oder abgewinkelten Handstückabschnitts 49 angeordnet.

Der Winkel W 4 zwischen der Längsmittelachse 1a und der Mittelachse des Werkzeugs 45 und der sie schneidenden Handstück-Mittelachse 1a ist stumpf und beträgt vorzugsweise etwa 95 bis 102°, insbesondere etwa 100°. Diese Form ist an die Kieferform des menschlichen Gebisses besonders günstig angepasst, und deshalb eignet sich diese Form insbesondere für ein dentalmedizinisches Handstück 1. Andererseits ergibt diese Form eine raumgünstige Anordnung zwischen dem Werkzeug 45 und der Bildaufnahmevorrichtung 31 mit einem verhältnismäßig großem Abstand zwischen diesen Teilen. Die Querschnittsgröße des vorzugsweise runden Handstückabschnitts 49 verringert sich nach vorne vorzugsweise kontinuierlich, wodurch der Blick des Behandlers auf die Behandlungsstelle verbessert wird.

Zwischen der Bildaufnahmevorrichtung 31 und der Einstecköffnung 47 kann wenigstens eine Auslassöffnung 51 für einen Medienauslass wie Luft oder Wasser oder Spray oder Licht vorgesehen sein, wobei sich die wenigstens eine andeutungsweise dargestellte Medienleitung 5 zu dieser Auslassöffnung 51 erstreckt.

Im übrigen ist dieses Ausführungsbeispiel hinsichtlich der Signal- bzw. Datumund/oder Bildverarbeitung entsprechend dem Ausführungsbeispiel nach Fig. 1 und 2 ausgebildet, so dass zur Vermeidung von Wiederholungen darauf verwiesen werden kann und eine wiederholte Beschreibung dieser Merkmale nicht erforderlich ist.

Letzteres gilt auch für das Ausführungsbeispiel nach Fig. 4, bei dem ebenfalls gleiche oder vergleichbare Teile mit gleichen Bezugszeichen versehen sind. Das Handstück 1 dieses Ausführungsbeispiels ist ebenfalls ein Arbeitshandstück, nämlich ein sog. Chirurgie-Handstück, das sich gerade erstreckt und die Einstecköffnung 47 für das Werkzeug 45 an seinem vorderen Ende nicht seitlich sondern stirnseitig aufweist, so dass das Werkzeug 45 sich nach vorne erstreckt. Auch bei diesem Ausführungsbeispiel kann ein hinterer, sich gerade erstreckender Abschnitt des Handstücks 1 einen Griffabschnitt 2 bilden, von dem sich nach vorne ein Halsabschnitt 3 vorzugsweise kontinuierlich verjüngend erstreckt. Es ist auch möglich, den Halsabschnitt 3 als Griffabschnitt bei einer Behandlung zu benutzen.

Auch bei diesem Ausführungsbeispiel weist das Display 21 den Abstand e vom vorderen Ende des Handstücks 1 auf. Es kann auch eine Bildaufnahmevorrichtung 31 dem Display 21 zugeordnet sein, die im vorderen Endbereich des Handstücks z.B. in einem seitlichen Materialansatz 33 integriert ist, wie es vorbeschrieben worden ist. Dabei kann es je nach Benutzung vorteilhaft sein, die Bildaufnahmevorrichtung 31 auf der dem Display 21 gegenüberliegenden Seite des Handstücks 1 anzuordnen, wodurch sich eine günstige Bildaufnahmeposition für die Bildaufnahmevorrichtung 31 in der Behandlungsposition des Handstücks 1 ergibt.

Es ist im übrigen möglich und vorteilhaft, das Handstück 1 jeweils rückseitig mit einem Kupplungselement auszubilden, das zu einer Steck/Drehkupplung 56 zum lösbaren Verbinden des Handstücks 1 mit einem Anschlussteil 57 passt (Fig. 3 und 4). Mit dem Anschlussteil 57 ist die Versorgungsleitung 8 verbunden. Die Steck/Drehkupplung 56 weist vorzugsweise eine hohlzylindrische Steckausnehmung 56a auf, in die ein passender Steckzapfen 56b einsteckbar ist. Zur Sicherung des Handstücks in der Kupplungsstellung ist der Steck/Drehkupplung 56 eine Verrastungsvorrichtung 58 zugeordnet, die manuell überdrückbar ist und beim Kuppeln bzw. Zusammenstecken selbsttätig elastisch einrastet und beim Trennen der Kupplung 56 überdrückt wird und selbsttätig ausrastet.

In den Fällen, in denen das Handstück 1 wenigstens eine Medienleitung 5 aufweist, durchsetzt diese die Steck/Drehkupplung 56 Z-förmig, wobei im Bereich des radialen Durchgangs der Medienleitung 5 durch die hohlzylindrische Teilungsfuge der Kupplung 56 eine Ringnut 56c im Steckzapfen 56b oder in der Steckausnehmung 56a angeordnet ist und dieser radiale Durchgang auf beiden Seiten durch jeweils einen Dichtring 56d abgedichtet ist, der in einer Ringnut des Steckzapfens oder der Steckausnehmung sitzt, wie es an sich ebenfalls bekannt ist. Eine solche Ausgestaltung ermöglicht ein Drehen des Handstücks 1 auch über 360° hinaus, wobei ein kontinuierlicher Durchgang in der Medienleitung 5 gewährleistet ist. In vergleichbarer Weise lässt sich mit einem Schleifring und einem zugehörigen Schleifkontakt im Bereich der hohlzylindrischen Teilungsfuge beim Drehen auch ein kontinuierlicher Leitungsdurchgang für die Stromversorgung und für die wenigstens eine Signalleitung 42 verwirklichen.

Für den Antrieb eines bewegbaren, z.B. drehbaren Werkzeugs 45 kann eine im vorderen Endbereich des Handstückabschnitts 49 angeordnete Turbine dienen, die durch Druckluft angetrieben wird, die in einer besonderen Druckleitung zuführbar ist. Im Falle einer Steck/Drehkupplung 56 durchsetzt diese Druckleitung die hohlzylindrische Teilungsfuge der Kupplung ebenfalls Z-förmig im vorbeschriebenen Sinne.

Das Werkzeug 45 kann auch durch eine im Handstück 1 drehbar gelagerte Antriebswelle antreibbar sein, die in ihrem hinteren Endbereich ein Kupplungselement aufweist, das beim Verbinden des Handstücks 1 mit dem Anschlussteil gleichzeitig mit einem Gegenkupplungselement verbunden wird, das durch einen im Antriebsteil angeordneten Antriebsmotor, insbesondere einen Elektromotor, antreibbar ist. Auch ein solcher Antrieb ist an sich bekannt und braucht deshalb nicht näher beschrieben zu werden.

Das Handstück 1 besteht jeweils mit seinen Einzelteilen aus korrosionsfestem Material, das auch gegen Desinfektions- und Sterilisationsmittel, z.B. auch eine entsprechende Desinfektions- bzw. Sterilisationstemperatur, unempfindlich ist. Dabei können die Teile aus korrosionsfestem Metall, insbesondere legiertem Stahl, oder Kunststoff bestehen, wobei ein Arbeitshandstück vorzugsweise aus Metall besteht und ein Funktions- oder Sondenhandstück vorzugsweise aus Kunststoff besteht.

Die Displayansichten gemäß Fig. 5 bis 7 zeigen arbeitsrelevante Daten oder Bilder des zugehörigen Handstücks 1 oder der Behandlungsstelle 14. In Fig. 5 ist eine Anzeige der Drehzahl vorgesehen, z.B. eine numerische Anzeige der Umdrehungen pro Zeiteinheit. Anstelle oder zusätzlich dieser Drehzahlanzeige kann eine Drehrichtungsanzeige vorgesehen sein, siehe den dargestellten Pfeil.

Fig. 6 zeigt ein Ausführungsbeispiel für die Anzeige der Kurve eines Diagramms, wobei die Kurve z.B. die jeweils vorhandene Leistung wiedergeben kann.

Fig. 7 zeigt eine Bildaufnahme von der Behandlungsstelle 14, hier von wenigstens einem Zahn 15, wobei der Behandler die gewünschte Behandlungsstelle, z.B. eine Kavität, erkennen kann. Diese Abbildung ist vorzugsweise so groß, dass nicht nur der zu behandelnde Zahn 15 sondern auch dessen Nachbarzähne zum Teil dargestellt sind.

Es ist im Rahmen der Erfindung auch möglich und unter Berücksichtigung einer möglichst kleinen Bauweise für das Handstück 1 und der damit verbundenen Raumknappheit vorteilhaft, die auf dem Display 21 anzuzeigenden Daten drahtlos zu übertragen. Hierzu ist dem Handstück 1 ein Datenempfänger 61 für drahtlose Datenübertragung und eine Datenumwandlungsvorrichtung 62 zugeordnet, die die übertragenen Daten in auf dem Display 21 anzeigbare Daten umwandelt. Dem Datenempfänger 61 können arbeitsrelevante Daten von einer Versorgungseinrichtung des zugehörigen Behandlungsstuhls übertragen werden. Es kann sich aber auch um Bilddaten handeln, die von einem Datensender 63 der Bildumwandlungsvorrichtung 36 dem Datenempfänger 61 zur Anzeige auf dem Display 21 drahtlos übertragen werden.

Unabhängig davon kann das Handstück 1 auch so ausgebildet sein, dass die Daten des Datensenders 63 einem dem Bildschirm 41 zugehörigen Datenempfänger drahtlos übertragbar sind und auf dem Bildschirm 41 anzeigbar sind.

Eine solche, die drahtlose Datenübertragung ermöglichende Ausgestaltung ist bei allen Ausführungsbeispielen möglich und vorteilhaft.

## Patentansprüche

1. Medizinisches oder zahnmedizinisches Handstück (1)
**dadurch gekennzeichnet,**
**dass** es ein Display (21) zur Anzeige von arbeitsrelevanten Daten oder Bildern aufweist.

2. Handstück nach Anspruch 1,
**dadurch gekennzeichnet,**
**dass** es stabförmig geformt ist und das Display (21) einen längs gerichteten Abstand (e) vom vorderen Ende des Handstücks (1) aufweist, der vorzugsweise wenigstens etwa 20 bis 80 mm, insbesondere etwa 30 bis 50 mm, beträgt oder größer ist.

3. Handstück nach Anspruch 2,
**dadurch gekennzeichnet,**
**dass** das Handstück (1) hinter dem Display (21) einen sich vorzugsweise gerade erstreckenden Griffabschnitt (3) aufweist.

4. Handstück nach einem der vorherigen Ansprüche,
**dadurch gekennzeichnet,**
**dass** der vordere Längsbereich des Handstücks (1) seitlich abgewinkelt oder gekrümmt ist.

5. Handstück nach Anspruch 4,
**dadurch gekennzeichnet,**
**dass** das Display (21) in einem Längsbereich angeordnet ist, der sich etwa vom vorderen Endbereich des Griffabschnitts (9; 48) bis etwa zum mittleren Bereich der Abwinklung oder Krümmung erstreckt.

6. Handstück nach Anspruch 4 oder 5,
**dadurch gekennzeichnet,**
**dass** das Display (21) auf der Seite des Handstücks (1) angeordnet ist, die der Abwinklungsrichtung oder Krümmungsrichtung abgewandt ist.

7. Handstück nach einem der vorherigen Ansprüche,
**dadurch gekennzeichnet,**
**dass** die Displayfläche (21a) an der Mantelfläche des Handstücks (1) angeordnet ist oder die Mantelfläche überragt und sich vorzugsweise dazu etwa parallel erstreckt oder mit der Mantelfläche einen spitzen Winkel (W3) einschließt, der nach vorne offen ist.

8. Handstück nach einem der vorherigen Ansprüche,
**dadurch gekennzeichnet,**
**dass** es ein oder mehrere Betätigungselemente (22) aufweist zum Ein -und Ausschalten der Anzeige unterschiedlicher Daten oder Bilder auf dem Display (21).

9. Handstück nach Anspruch 8,
**dadurch gekennzeichnet,**
**dass** das oder die Betätigungselemente (22) im Randbereich des Displays (21) angeordnet sind, vorzugsweise im hinteren Randbereich und/oder im vorderen Randbereich.

10. Handstück nach Anspruch 8 oder 9,
**dadurch gekennzeichnet,**
**dass** ein Betätigungselement (22) vorgesehen ist, mit dem ein Standbild auf dem Display (21) ein- und ausschaltbar ist und/oder ein Betätigungselement vorgesehen ist, mit dem eine Bildwiedergabe an einem dem Behandlungsplatz zugeordneten Monitor (41) ein- und ausschaltbar ist.

11. Handstück, insbesondere nach einem der vorherigen Ansprüche,
**dadurch gekennzeichnet,**
**dass** es in seinem vorderen Endbereich eine Bildaufnahmevorrichtung (31) aufweist, deren Bilder auf dem Display (21) anzeigbar sind.

12. Handstück nach Anspruch 11,
**dadurch gekennzeichnet,**
**dass** die Bildaufnahmevorrichtung (31) eine Lichteinlassöffnung (34) aufweist, die durch eine lichtdurchlässige Verschlussscheibe (35) abgedeckt ist.

13. Handstück nach Anspruch 11 oder 12,
**dadurch gekennzeichnet,**
**dass** die Bildaufnahmevorrichtung (31) ein Kameramodul (32) aufweist.

14. Handstück nach einem der Ansprüche 11 bis 13,
**dadurch gekennzeichnet,**
**dass** die Bildaufnahmevorrichtung (31) außermittig im Randbereich des Handstücks (1) angeordnet ist.

15. Handstück nach einem der Ansprüche 11 bis 14,
**dadurch gekennzeichnet,**
**dass** die Bildaufnahmevorrichtung (31) in der Abwinklungs- oder Krümmungsebene des Handstücks (1) angeordnet ist, vorzugsweise auf der nach vorne gerichteten Seite der Abwinklung oder Krümmung angeordnet ist.

16. Handstück nach einem der vorherigen Ansprüche 11 bis 15,
**dadurch gekennzeichnet,**
**dass** die Bildaufnahmevorrichtung (12) in einem Materialansatz (33) angeordnet ist, der vorzugsweise seitlich angeordnet ist.

17. Handstück nach einem der vorherigen Ansprüche,
**dadurch gekennzeichnet,**
**dass** es ein Funktionshandstück und/oder Sondenhandstück und/oder ein Arbeitshandstück ist.

18. Handstück nach einem der vorherigen Ansprüche 11 bis 16,
**dadurch gekennzeichnet,**
**dass** der Abstand der Bildaufnahmevorrichtung (31) vom vorderen Ende der Auslassdüse (4) weniger als 30 mm beträgt, vorzugsweise etwa 5 mm bis 10 mm beträgt.

19. Handstück nach einem der vorherigen Ansprüche 11 bis 17,
**dadurch gekennzeichnet,**
**dass** bei einem Arbeitshandstück (1) mit einer seitlich offenen Einstecköffnung (46) für ein Werkzeug (45) in einem Kopf (13) des Handstücks (1) die Bildaufnahmevorrichtung (31) im vorderen Endbereich vor der hinter dem Kopf (13) auf der Seite angeordnet ist, auf der sich die Einstecköffnung (46) befindet, und schräg auf die Mittelachse der Einstecköffnung (46) gerichtet ist (Fig. 3).

20. Handstück nach einem der vorherigen Ansprüche 11 bis 17,
**dadurch gekennzeichnet,**
**dass** bei einem Arbeitshandstück (1) mit einer nach vorne offenen Einstecköffnung (46) für ein Werkzeug (45) die Bildaufnahmevorrichtung (31) im vorderen Endbereich des Handstücks (1) angeordnet ist, insbesondere im seitlichen Umfangsbereich des Handstücks (1) und nach vorne gerichtet ist (Fig. 4).

21. Handstück nach einem der vorherigen Ansprüche,
**dadurch gekennzeichnet,**
**dass** es einen dem Display zugehörigen Datenempfänger zum Empfang von drahtlos übertragenen Daten aufweist, die auf dem Display (21) anzeigbaren arbeitsrelevanten Daten oder Bildern entsprechen.

22. Handstück nach einem der vorherigen Ansprüche 11 bis 21,
**dadurch gekennzeichnet,**
**dass** des einen der Bildaufnahmevorrichtung (31) zugehörigen Datensender (63) zum Senden von den aufgenommenen Bildern entsprechenden Daten aufweist.
